(19) Europäisches Patentamt
European Patent Office
Office européen des brevets

(11) Veröffentlichungsnummer : **0 132 731**
**B1**

# (12) EUROPÄISCHE PATENTSCHRIFT

(45) Veröffentlichungstag der Patentschrift : **30.12.86**

(51) Int. Cl.⁴ : **C 07 C 143/42**, C 07 C 139/12

(21) Anmeldenummer : **84108292.8**

(22) Anmeldetag : **14.07.84**

(54) **Chromotropsäure-tetraalkalisalze, Verfahren zu ihrer Gewinnung und ihre Verwendung.**

(30) Priorität : **28.07.83 DE 3327275**

(43) Veröffentlichungstag der Anmeldung : **13.02.85 Patentblatt 85/07**

(45) Bekanntmachung des Hinweises auf die Patenterteilung : **30.12.86 Patentblatt 86/52**

(84) Benannte Vertragsstaaten : **CH DE FR GB IT LI**

(56) Entgegenhaltungen : **DE-C- 67 563**

(73) Patentinhaber : **BAYER AG**
**Konzernverwaltung RP Patentabteilung**
**D-5090 Leverkusen 1 Bayerwerk (DE)**

(72) Erfinder : **Hammerschmidt, Erich, Dr.**
**Schützenstrasse 25**
**D-5060 Bergisch-Gladbach 2 (DE)**
Erfinder : **Behre, Horst, Dr.**
**Zur alten Linde 12**
**D-5068 Odenthal (DE)**
Erfinder : **Blank, Heinz Ulrich, Dr.**
**Am Geusfelde 35**
**D-5068 Odenthal (DE)**
Erfinder : **Mayer, Dietmar, Dr.**
**In den Wiesen 1**
**D-5060 Bergisch-Gladbach (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft Tetraalkalisalze der Chromotropsäure in kristalliner Form, ein Verfahren zu ihrer Gewinnung und ihre Verwendung zur Herstellung reiner Chromotropsäure (1,8-Dihydroxy-naphthalin-3,6-disulfonsäure) in Form ihrer Dialkalisalze.

Chromotropsäure ist ein wichtiges Zwischenprodukt zur Herstellung von Farbstoffen (siehe Ullmann's Enzyklopädie der Technischen Chemie, 4. Auflage, Band 17, S. 97).

Chromotropsäure bzw. ihre Dialkalisalze werden bislang nach zwei verschiedenen Verfahren hergestellt (siehe Ullmann's Enzyklopädie loc. cit.). Nach Verfahren (1) wird T-Säure (1-Amino-naphthalin-3,6,8-trisulfonsäure) durch alkalische Druckhydrolyse mit wäßriger Natronlauge in Chromotropsäure überführt. Nach Verfahren (2) wird T-Säure zunächst durch saure Druckhydrolyse mit verdünnter Schwefelsäure in 1-Naphthol-3,6,8-trisulfonsäure überführt und diese in einem zweiten Reaktionsschritt drucklos mit konzentrierter Natronlauge zu Chromotropsäure umgesetzt. Die Chromotropsäure wird bei beiden Verfahren üblicherweise durch Ansäuern der alkalischen Reaktionsgemische und Aussalzen mit Kochsalz in Form ihres Dinatriumsalzes isoliert.

Die beiden Verfahren liefern eine Chromotropsäure unterschiedlicher Qualität (Reinheit). Ein Maß für die Qualität der Chromotropsäure ist ihr Gehalt an diazotierbaren Verbindungen ; je geringer dieser Gehalt umso besser die Qualität der Chromotropsäure. Gemäß Verfahren (1) wird eine Chromotropsäure minderer Qualität erhalten (Chromotropsäure C) ; ihr Gehalt an diazotierbaren Verbindungen liegt über 6 Mol-%. Gemäß Verfahren (2) wird eine Chromotropsäure hoher Qualität erhalten (Chromotropsäure 4G) ; ihr Gehalt an diazotierbaren Verbindungen beträgt höchstens 3 Mol-%. In bezug auf die Reinheit der anfallenden Chromotropsäure wäre daher das Verfahren (2) das günstigere Verfahren. Verfahren (2) hat jedoch den Nachteil, daß es als Zweistufen-Verfahren aufwendiger und teurer ist, eine längere Reaktionszeit erfordert und daß bei der sauren Druckhydrolyse erhebliche Korrosionsprobleme auftreten.

Es wurde nun überraschenderweise gefunden, daß man auch nach Verfahren (1) eine qualitativ hochwertige Chromotropsäure (Chromotropsäure 4G) erhalten kann, wenn man die gemäß Verfahren (1) erhaltene Chromotropsäure nicht wie bislang als Dialkalisalz, sondern zunächst als Tetraalkalisalz isoliert und dieses durch Ansäuren in das Dialkalisalz überführt.

Die Tetraalkalisalze der Chromotropsäure wurden bislang noch nicht in fester kristalliner Form erhalten und galten als nicht isolierbar (siehe die entsprechenden Angaben in DE-PS 67 563, Seite 5, linke Spalte). Überraschenderweise wurde gefunden, daß sich diese Tetraalkalisalze der Chromotropsäure bei Einhaltung bestimmter Bedingungen sehr wohl in fester kristalliner Form isolieren lassen, daß die Chromotropsäure-tetraalkalisalze sogar in groben, gut abtrennbaren Kristallen kristallisieren und daß die Tetraalkalisalze überraschenderweise bei ihrer Fällung sehr viel weniger von den die Chromotropsäure stets begleitenden Verunreinigungen (Aminonaphthalin- und Amino-hydroxy-naphthalin-mono-, -di-oder -trisulfonsäuren) mitreißen und deshalb in einer sehr viel reineren Form anfallen als die Dialkalisalze der Chromotropsäure.

Die Erfindung betrifft daher Chromotropsäure-tetraalkalisalze der Formel

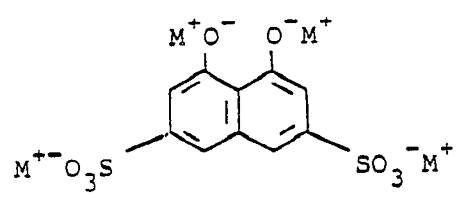

in der M für Natrium oder Kalium steht, in fester kristalliner Form, ein Verfahren zur Gewinnung dieser festen kristallinen Chromotropsäure-tetraalkalisalze und ihre Verwendung zur Herstellung reiner Chromotropsäure in Form ihrer Dialkalisalze.

Das Verfahren zur Gewinnung der Chromotropsäure-tetraalkalisalze in fester kristalliner Form ist dadurch gekennzeichnet, daß man alkalische Chromotropsäure-Lösungen, deren pH-Wert mindestens 8 beträgt, aufkonzentriert und/oder mit entsprechenden Alkali-Ionen sättigt und die Lösungen auf Temperaturen unter 70 °C abkühlt.

Der pH-Wert der alkalischen Lösungen beträgt vorzugsweise mindestens 9,5.

Die alkalischen Lösungen werden zweckmäßig auf Chromotropsäure-Konzentrationen von 0,5 bis 2,0 Mol Chromotropsäure je Liter Lösung, vorzugsweise 0,7 bis 1,5 Mol Chromotropsäure je Liter Lösung, aufkonzentriert.

Zur Isolierung werden die alkalischen Lösungen der Chromotropsäure vorzugsweise auf Temperaturen unter 30 °C abgekühlt.

Zur Verbesserung der Isolierausbeute können Alkali-Ionen in Form ihrer Salze bis zur Sättigungsgrenze zugesetzt werden. Als geeignete Salze seien beispielsweise Natrium- oder Kalium-Halogenide, insbesondere die -Chloride, ferner die -Sulfate, -Phosphate, -Carbonate, -Nitrate, -Nitrite oder -Azetate genannt.

Als Ausgangsprodukte für die erfindungsgemäße Gewinnung der Chromotropsäure-tetraalkalisalze eignen sich z. B. die alkalischen Lösungen, wie sie bei der alkalischen Druckhydrolyse von T-Säure (siehe Verfahren (1) zur Herstellung von Chromotropsäure) oder bei der drucklosen Umsetzung von 1-Naphthol-3,6,8-trisulfonsäure mit konzentrierter Natronlauge (siehe Verfahren (2) zur Herstellung von Chromotropsäure) anfallen. Ferner können die alkalischen Lösungen eingesetzt werden, wie sie beim Auflösen von Chromotropsäure-dialkalisalzen beliebiger Qualität in Natronlauge erhalten werden.

Bei der erfindungsgemäßen Verwendung der erfindungsgemäßen Chromotropsäure-tetraalkalisalze zur Herstellung reiner Chromotropsäure wird das feste kristalline Tetraalkalisalz durch Anschlämmen in Wasser und Ansäuern der Suspension in das Chromotropsäure-dialkalisalz überführt.

Die festen kristallinen Chromotropsäure-tetraalkalisalze eignen sich auch als Ausgangsprodukte für die Herstellung von Chromotropsäure-Folgeprodukten wie die Ethyl-Chromotropsäure.

### Beispiel 1

Das bei der alkalischen Druckhydrolyse (15 Stunden bei 200 °C) von 1 Mol T-Säure (Dinatriumsalz) mit 1 885 g 10 %iger Natronlauge anfallende und vom Ammoniak befreite Reaktionsgemisch wird auf ein Gesamtvolumen von 650 ml eingeengt (Gehalt der Lösung an Chromotropsäure 1,2 Mol/l) und auf 30 °C abgekühlt.

Das ausgefallene Chromotropsäure-tetranatriumsalz wird abgesaugt und mit 250 ml gesättigter Kochsalzlösung gewaschen.

Ausbeute : 68 % der Theorie Chromotropsäure, bezogen auf eingesetzte T-Säure.

Das Tetranatriumsalz wird in 600 ml Wasser angeschlämmt und mit 300 ml 30 %iger Salzsäurelösung in das Dinatriumsalz der Chromotropsäure überführt.

Ausbeute an Dinatriumsalz : 67 % der Theorie, bezogen auf T-Säure.

Gehalt an diazotierbaren Verbindungen : 1,8 Mol-%.

### Beispiel 2

488 g Chromotropsäure C (Dinatriumsalz ; Gehalt an diazotierbaren Verbindungen : 11,3 Mol-%) werden in der Siedehitze in 1 500 ml 10 %iger Natronlauge gelöst. Die Lösung wird mit 300 g Kochsalz versetzt und anschließend auf 20 °C abgekühlt. Das sich abscheidende Tetranatriumsalz wird abgesaugt und mit gesättigter Kochsalzlösung gewaschen.

Anschließend wird das Tetranatriumsalz in 1 000 ml Wasser suspendiert und durch Zusatz von 400 ml 30 %iger wäßriger Salzsäure in das Chromotropsäure-dinatriumsalz umgewandelt.

Die Ausbeute an Chromotropsäure-dinatriumsalz beträgt 81 % der Theorie, bezogen auf die eingesetzte Chromotropsäure C.

Der Gehalt des gereinigten Chromotropsäure-dialkalisalzes an diazotierbaren Verbindungen beträgt nur noch 2,3 Mol-%.

## Patentansprüche

1. Chromotropsäure-tetraalkalisalze der Formel

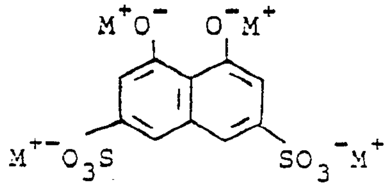

in der M für Natrium oder Kalium steht, in fester kristalliner Form.

2. Verfahren zur Gewinnung der Chromotropsäure-tetraalkalisalze in fester kristalliner Form, dadurch gekennzeichnet, daß man alkalische Chromotropsäure-Lösungen, deren pH-Wert mindestens 8 beträgt, aufkonzentriert und/oder mit entsprechenden Alkali-Ionen sättigt und die Lösungen auf Temperaturen unter 70 °C abkühlt.

3. Verfahren gemäß Anspruch 2, dadurch gekennzeichnet, daß der pH-Wert der alkalischen Chromotropsäure-Lösungen mindestens 9,5 beträgt.

4. Verfahren gemäß Ansprüchen 2 oder 3, dadurch gekennzeichnet, daß man die alkalischen Chromotropsäure-Lösungen auf einen Gehalt von 0,5 bis 2,0 Mol Chromotropsäure/l Lösung aufkonzentriert.

5. Verfahren gemäß Ansprüchen 2 oder 3, dadurch gekennzeichnet, daß man die alkalischen Chromotropsäure-Lösungen auf einen Gehalt von 0,7 bis 1,5 Mol Chromotropsäure/l Lösung aufkonzentriert.

6. Verfahren gemäß Ansprüchen 2, 3, 4 oder 5, dadurch gekennzeichnet, daß man die alkalischen Lösungen auf Temperaturen unter 30 °C abkühlt.

7. Verwendung der festen kristallinen Chromotropsäuretetraalkalisalze zur Herstellung reiner Chromotropsäure in Form ihrer Dialkalisalze.

## Claims

1. Tetra-alkali metal salts of chromotropic acid of the formula

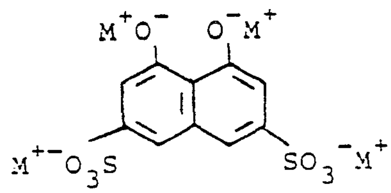

in which M represents sodium or potassium, in solid crystalline form.

2. Process for the recovery of the tetra-alkali metal salts of chromotropic acid in solid crystalline form, characterised in that alkaline solutions of chromotropic acid which have a pH value of at least 8 are concentrated and/or saturated with corresponding alkali metal ions and the solutions are cooled to temperatures below 70 °C.

3. Process according to Claim 2, characterised in that the pH value of the alkaline solutions of chromotropic acid is at least 9.5.

4. Process according to Claims 2 or 3, characterised in that the alkaline solutions of chromotropic acid are concentrated to a content of 0.5 to 2.0 moles of chromotropic acid/l of solution.

5. Process according to Claims 2 or 3, characterised in that the alkaline solutions of chromotropic acid are concentrated to a content of 0.7 to 1.5 moles of chromotropic acid/l of solution.

6. Process according to Claims 2, 3, 4 or 5, characterised in that the alkaline solutions are cooled to temperatures below 30 °C.

7. Use of the solid crystalline tetra-alkali metal salts of chromotropic acid for the preparation of pure chromotropic acid in the form of its di-alkali metal salts.

## Revendications

1. Sels tétra-alcalins de l'acide chromotropique de formule :

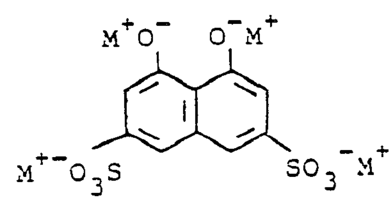

dans laquelle M représente le sodium ou le potassium, à l'état solide cristallisé.

2. Procédé pour obtenir les sels tétra-alcalins de l'acide chromotropique à l'état solide cristallisé, caractérisé en ce que l'on concentre des solutions alcalines de l'acide chromotropique qui ont un pH d'au moins 8 et/ou on les sature des ions alcalins correspondants et on refroidit les solutions à des températures inférieures à 70 °C.

3. Procédé selon la revendication 2, caractérisé en ce que le pH des solutions alcalines d'acide chromotropique est d'au moins 9,5.

4. Procédé selon les revendications 2 ou 3, caractérisé en ce que l'on concentre les solutions alcalines de l'acide chromotropique jusqu'à une teneur de 0,5 à 2,0 moles d'acide chromotropique par litre.

5. Procédé selon les revendications 2 ou 3, caractérisé en ce que l'on concentre les solutions alcalines de l'acide chromotropique jusqu'à une teneur de 0,7 à 1,5 mole d'acide chromotropique par litre.

6. Procédé selon les revendications 2, 3, 4 ou 5, caractérisé en ce que l'on refroidit les solutions alcalines à des températures inférieures à 30 °C.

7. Utilisation des sels tétra-alcalins de l'acide chromotropique à l'état solide cristallisé pour la préparation de l'acide chromotropique à l'état de sel di-alcalin.